# EUROPEAN PATENT SPECIFICATION

(11) **EP 2 235 736 B1**
(45) Date of publication and mention of the grant of the patent: **01.04.2015**
(21) Application number: 08859342.1
(22) Date of filing: 15.12.2008
(51) Int. Cl.: G01N 21/64, G01N 15/14

(54) **PATHOGEN DETECTION BY SIMULTANEOUS SIZE/FLUORESCENCE MEASUREMENT**
PATHOGENDETEKTION DURCH GLEICHZEITIGE GRÖSSEN-/FLUORESZENZMESSUNG
DÉTECTION DE PATHOGÈNES PAR MESURE DE TAILLE/FLUORESCENCE SIMULTANÉE

(30) Priority: 13.12.2007 US 13607
(43) Date of publication of application: 06.10.2010
(73) Proprietor: Azbil Corporation, Chiyoda-ku Tokyo 100-6419 (JP)
(72) Inventor: BINNIE, Erik, H., Tucson Arizona 85704 (US); MORRIS, Gregory, Scott, Tucson Arizona 85719 (US)
(74) Representative: Tischner, Oliver
(86) International application number: PCT/US2008/086886
(87) International publication number: WO 2009/076678

(56) References cited:
- EP-A2- 0 515 211
- WO-A2-2005/029046
- WO-A2-2006/073492
- US-A- 5 540 494
- US-A1- 2002 045 190
- US-A1- 2002 118 362
- US-A1- 2003 098 422
- US-A1- 2004 159 799
- US-B1- 6 787 104
- US-B2- 6 831 279
- US-B2- 6 885 440
- "Instantaneous Microbial Detection", , 1 January 2005 (2005-01-01), pages 1-6, XP55002158, Retrieved from the Internet: URL:http://www.biovigilant.com/PDFs/IMD%20 for%20Pharmamaceutical%20Mfg%2005-05-08.pd f [retrieved on 2011-07-07]
- "Why Simultaneous Sizing and Fluorescence is Necessary in Order to Do Microbial Detection", , 1 January 2005 (2005-01-01), XP55002196, Retrieved from the Internet: URL:http://www.biovigilant.com/PDFs/Why%20 Simultaneous%20Sizing%20and%20Fluorescence %20is%20Necessary%20for%20Microbial%20Dete ction%209-8-05-c.pdf [retrieved on 2011-07-07]

## Description

The present invention relates generally to a method for detecting airborne or liquid borne particles, and more particularly to a method for detecting airborne or liquid borne particles and classifying the detected particles. The invention has particular utility in detecting and classifying biological particles or contamination in clean environments such as aseptic manufacturing facilities, as well as other environments and will be described in connection with such utilities, although other utilities are contemplated.

The monitoring for environmental contamination, including biological particles, is important in a number of industrial and commercial environments such as manufacturing facilities for pharmaceuticals, food and hospitals, and has also become important in public spaces such as airports, banks, postal handling facilities and government offices where there is concern for possible urban terrorist attacks.

In the pharmaceutical, healthcare and food industries a real time detector of environmental biological particle levels is useful for public health, quality control and regulatory purposes. For example, parenteral drug manufacturers are required by the Food and Drug Administration to monitor the particulate and microbial levels in their aseptic clean rooms. Conventional microbiological methods require the collection of samples on growth media, and incubation at the correct temperature for the correct period of time (typically days). These methods assume that a viable microorganism is one that will undergo cellular division when placed in or on a growth media. For quantitative tests, growth is demonstrated by a visually detectable colony. There is a significant quantity of published literature that shows substantial limitations of using traditional culture and plate counting methods. For example, the published literature indicates variable results can be obtained depending upon the growth media used, the incubation time and temperature, and the condition of the microorganism prior to attempts to cultivate (e.g., slow growing, stressed, or sub-lethally damaged). Conventional methods also have no ability in real-time to locate probable sources of the contamination. In these applications, an instrument that can detect microbial particles, including bacteria, yeasts and molds, in the environment instantaneously and at low concentrations will be a useful tool and have significant advantages over conventional nutrient plate culture methods that require days for microbes to grow and be visually detected. It would also be useful to have an instrument that would be able to assist in locating, preferably in real-time, sources of particulate contamination.

There exist various prior art devices that employ particle size measurement and light induced fluorescence techniques as early warning sensors for bio-agents. Among these devices are Biological Agent Warning Sensor (BAWS) developed by MIT Lincoln Laboratory, fluorescence biological particle detection system of Ho (Jim Yew-Wah Ho, U.S. Patent Nos. 5,701,012; 5,895,922; 6,831,279); FLAPS and UV-APS by TSI of Minnesota (Peter P. Hairston; and Frederick R. Quant; US Patent No. 5,999,250), and a fluorescence sensor by Silcott (U.S. Patent No. 6,885,440). A proposed bio-sensor based on laser-induced fluorescence using a pulsed UV laser is described by T.H. Jeys, et al., Proc. IRIS Active Systems, vol. 1, p.235, 1998. This is capable of detecting an aerosol concentration of five particles per liter of air, but involves expensive and delicate instruments. Other particle counters are manufactured by Met One Instrument, Inc, of Grants Pass, Oregon, Particle Measurement Systems, Inc., of Boulder, Colorado, and Terra Universal Corp., of Anaheim, California.

Various detectors have been designed to detect airborne allergen particles and provide warning to sensitive individuals when the number of particles within an air sample exceeds a predetermined minimum value. Among these detectors are those described in U.S. Patent Nos. 5,646,597, 5,969,622, 5,986,555, 6,008,729, 6,087,947, and 7,053,783, all to Hamburger et al. These detectors all involve direction of a light beam through a sample of environmental air such that part of the beam will be scattered by any particles in the air, a beam blocking device for transmitting only light scattered in a predetermined angular range corresponding to the predetermined allergen size range, and a detector for detecting the transmitted light.

EP 0 515 211 A2 relates to an apparatus and method for phase resolved fluorescence lifetimes of independent and varying amplitude pulses.

US 2002/0045190 A1 concerns encoding methods using upconverting phosphors for high-throughput screening of catalysts. In particular an apparatus is shown, which is simultaneously detecting forward scattered light and the fluorescence of particles.

US 2004/0159799 A1 discloses an apparatus and method for detecting particles. A flow tube is surrounded by eight off-axis ellipsoidal mirrors all having a common first focus coincident with a portion of the flow tube and having a distinct second focus.

US 2003/0098422 A1 and WO 2005/029046 A3 disclose both a method and system for detecting classifying biological particles, by detecting scattered light and fluorescence of particles.

US 2002/0118362 A1 discloses a flow cytometer comprising a flow tube surrounded by a pair of off-axis ellipsoidal mirrors.

For the purpose of detection of biological particles, including microbes, in air or water, it is of importance to devise an effective system to measure both particle size and fluorescence generated intrinsically by the microbes. A prior application, commonly owned by the assignee of the present application, improves upon previous designs by providing a sensor system that is capable of simultaneously measuring particle size and detecting the presence of intrinsic fluorescence from metabolites and other bio-molecules, on a particle-by-particle basis. This prior art example comprises three main components: (1) a first optical system for measuring an individual particle size; (2) a second optical system to detect laser-induced intrinsic fluorescence signal from an individual particle; and (3) a data recording format for assigning both particle size and fluorescence intensity to an individual particle, and computer readable program code for differentiating microbes from non-microbes (e.g. inert dust particles).

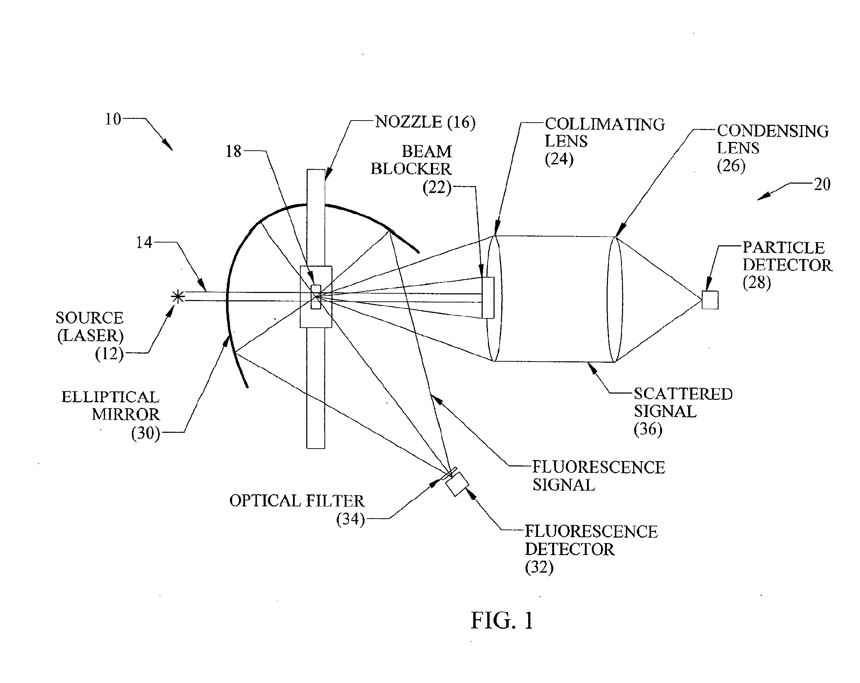

As shown in Figure 1, the prior art system 10 includes a excitation source 12, such as a laser, LED or other light source, providing a beam of electromagnetic radiation 14 having a source wavelength. The excitation source is selected to have a wavelength capable of exciting intrinsic fluorescence from metabolites inside microbes. Environmental air (or a liquid sample) is drawn into the system through a nozzle 16 for particle sampling. Nozzle 16 has an opening 18 in its middle section (forming a sample cell) to allow the laser beam to pass through the particle stream. Directly downstream from the laser beam is a Mie scattering particle-size detector 20. Mie scattering particle-size detector 20 includes a beam blocker 22 in front of a collimator lens 24, and a condenser lens 26 for focusing a portion of the light beam 14 scattered by particles in the sample stream onto a particle detector 28. Off axis from the laser beam 14, an elliptical mirror 30 is placed at the particle-sampling region in such a way that the intersection of the incoming particle stream and the laser beam is at one of the two foci of the ellipsoid, while a fluorescence detector 32 occupies the other focus. In this optical design, the elliptical mirror 30 concentrates the fluorescence signal from biological particles and focuses it onto the fluorescence detector 32. An optical filter 34 is placed in front of the fluorescence detector to block scattered light and pass the induced fluorescence. WO 2006/073492 A1 discloses such a system.

This system, however, has limitations in that the amount of fluorescence signal received by the fluorescence detector is small. The amount of noise accompanying this weak fluorescence signal makes it difficult to adequately process and amplify the data. Thus, there is a need for an improved design that efficiently gathers a greater amount of the fluorescence signal and allows a clearer fluorescence signal to be processed.

The present invention provides a method according to claim 1 which is capable of simultaneously measuring particle size and detecting the presence of intrinsic fluorescence from metabolites and other bio-molecules, on a particle-by-particle basis. The advantages of this detection scheme over the prior art are several. For one, it allows detailed analyses of data collected on each individual particle for characterizing the particle, such as intensity of fluorescence signal from a particle as a function of its cross-sectional area or volume, for the purpose of determining the biological status of the particles. Secondly, the present invention collects a greater amount of the fluorescence signal from a given particle, increasing the ability of the system to correctly identify biological particles.

The current invention comprises three main components: (1) a first optical system for measuring an individual particle size; (2) a second optical system to detect laser-induced intrinsic fluorescence signals from individual particles; and (3) a data recording format for assigning both particle size and fluorescence intensity to an individual particle, and computer readable program code for differentiating biological particles from non-biological particles (e.g. inert dust particles).

The present invention improves function of the second optical system by using a pair of elliptical mirrors with a pair of fluorescence detectors. The mirrors and detectors are positioned to collected fluorescence emission from the same particle as it is being measured for size. For each of the elliptical mirrors, one foci is at the intersection of the excitation light beam and one foci lies at the apex of the opposite facing elliptical mirror, where the fluorescence signal enters one of the fluorescence detectors. In a comparative example, an elliptical mirror and a spherical mirror are positioned to collect fluorescence emission from particles.

Further features and advantages of the present invention will be seen from the following detailed description, taken in conjunction with the accompanying drawings, wherein:
Figure 1 is a schematic diagram of an optical system in accordance with the commonly owned prior art.
Figure 2. is a plan view of the optical system in accordance with the present invention, for performing simultaneous measurements of particle size and fluorescence.
Figure 3 is a front view of the optical system of Figure 2.
Figure 4 is a top view of the optical system of Figure 2.
Figure 5 is a sectioned view of the optical system, taken along section A-A of Figure 4.
Figure 6 is a sectioned view of the optical system, taken along section B-B of Figure 4.
Figure 7 illustrates an alternative embodiment of the invention.
Figure 8 illustrates an alternative example comprising an elliptical and a spherical mirror.
Figure 9 is a block diagram of a measurement scheme in accordance with an embodiment of the present invention.

The methods of the present invention can be used to detect and classify particles in liquids or gases by simultaneously measuring the size and any intrinsic fluorescence from the particles. The methods of the present invention may further be used to differentiate and/or classify biological particles from inert particles.

The present invention is a method using an optical system for a fluid particle detector system. This system is designed, for example, to detect airborne or liquid borne particles, such as biologic particles, in air or liquid media in industrial applications such as the food and pharmaceutical manufacturing industries and hospitals, as well as clean room and other controlled environment applications. The present invention may also be used in other applications, for example in buildings or in public transportation areas, to detect harmful levels of other airborne or liquid borne particles that may exist naturally, such as mold or bacteria, or which may have been accidentally, inadvertently or deliberately released. The systems of the present invention also may be used to detect bio-terrorist agents deliberately released by terrorists or others.

The term "fluid borne particles" as used herein means both airborne particles and liquid borne particles. Liquid borne particles include those in water or other liquid media. Fluid borne particles also includes those in air and other gases. As used here in "waterborne particles" include those in water and in liquids comprising water.

The term "microbial" particle, "biological" particle" or "biological" agent is defined as any microorganism, pathogen, or infectious substance, biological toxin, or any naturally occurring, bioengineered or synthesized component of any such microorganism, pathogen, or infectious substance, whatever its origin or method of production. Such biological agents include, for example, biological toxins, bacteria, viruses, rickettsiae, spores, fungi, and protozoa, as well as others known in the art.

The term "pathogen" as used herein refers to any airborne or liquid borne particle, biological agent or toxin, which could potentially harm or even kill humans exposed to such particles if present in sufficient quantities.

"Biological toxins" are poisonous substances produced or derived from living plants, animals or microorganisms, but also can be produced or altered by chemical means. A toxin, however, generally develops naturally in a host organism (i.e., saxitoxin is produced by marine algae), but genetically altered and/or synthetically manufactured toxins have been produced in a laboratory environment. Compared with microorganisms, toxins have a relatively simple biochemical composition and are not able to reproduce themselves. In many aspects, they are comparable to chemical agents. Such biological toxins are, for example, botulinum and tetanus toxins, staphylococcal enterotoxin B, tricothocene mycotoxins, ricin, saxitoxin, Shiga and Shiga-like toxins, dendrotoxins, erabutoxin b, as well as other known toxins.

The detector system of the present invention is designed to detect airborne or liquid borne particles and produce outputs indicating, for instance, the number of particles of each size within the range, which is detected in a sample, and indicate whether the particles are biologic or non-biologic. The system also may produce an alarm signal or other response if biological particles are detected and/or if the number of particles exceeds a predetermined threshold value, for example the number of detected particles is above a normal background level.

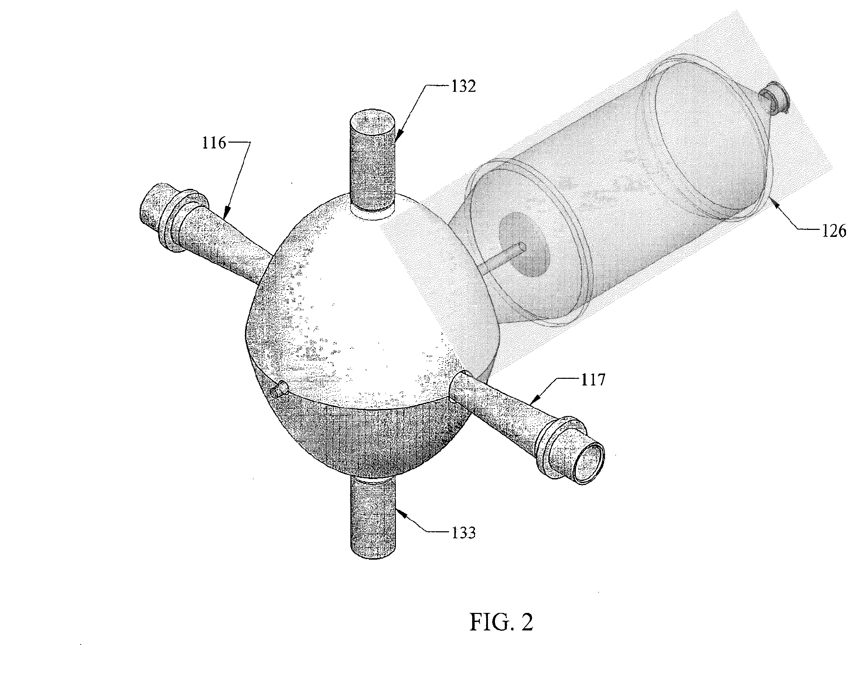

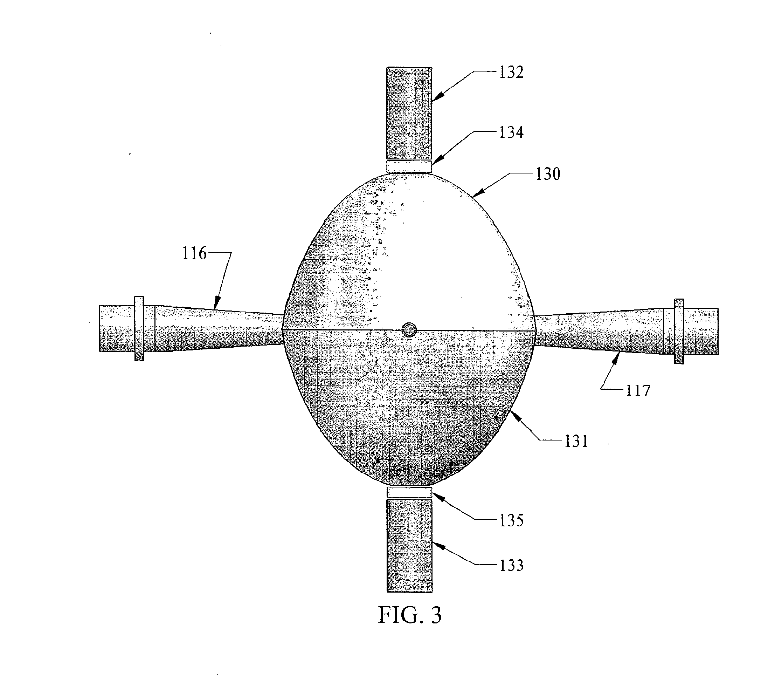

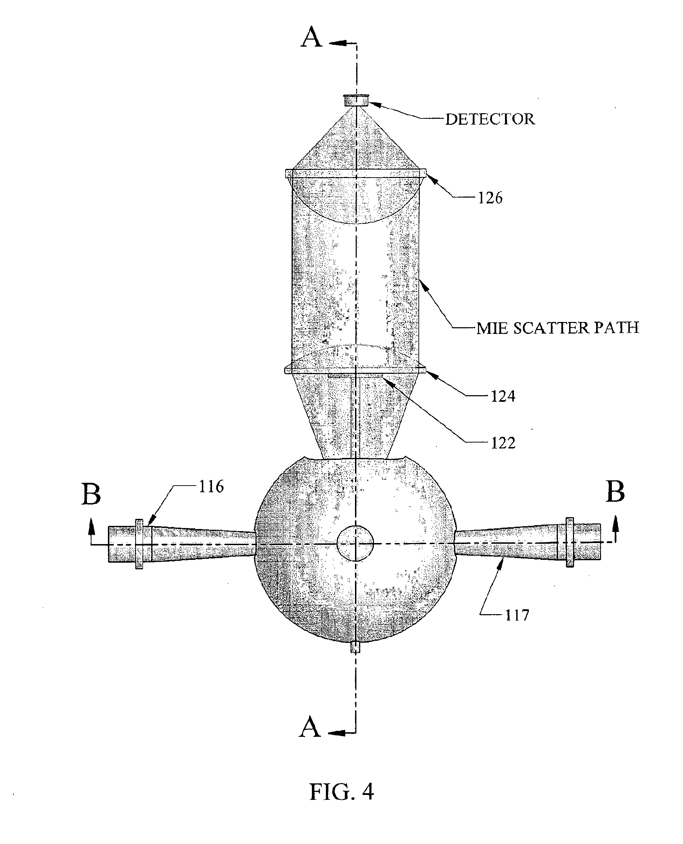

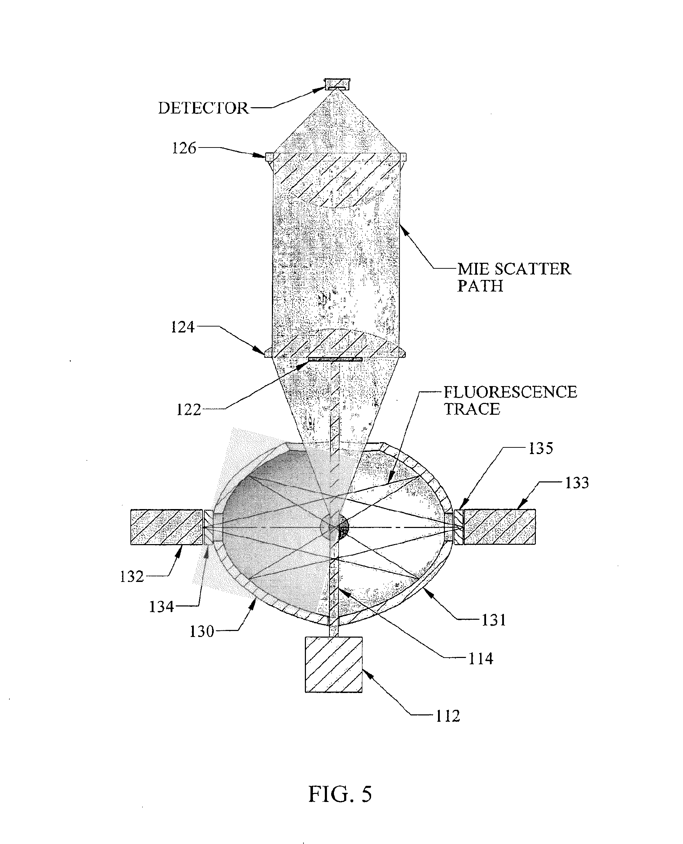

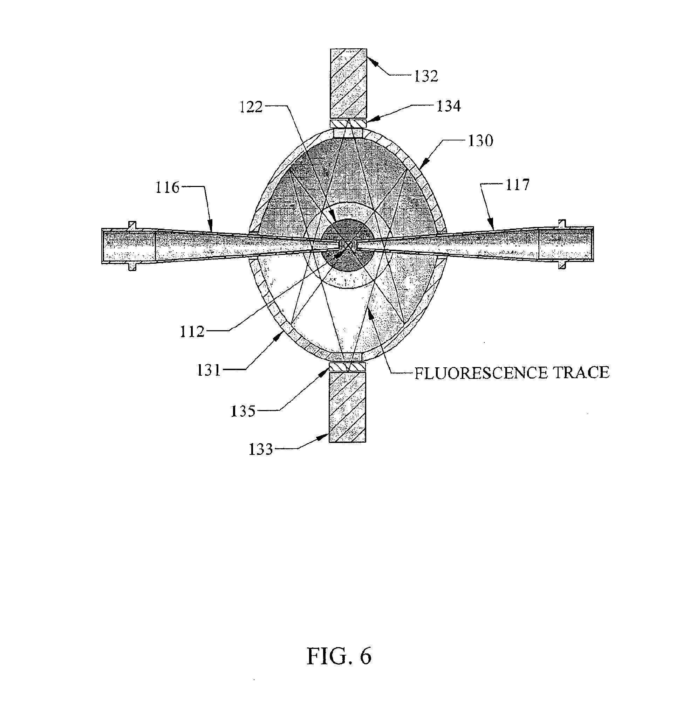

Figures 2-6 are illustrations of a preferred embodiment for a fluid particle detector system according to the present invention. As shown in Figures 5 and 6, the system includes an excitation source 112 such as a laser providing a beam of electromagnetic radiation 114 having a source wavelength. The excitation source is selected to have a wavelength capable of exciting intrinsic fluorescence from metabolites inside microbes and biological particles. The excitation source is also chosen to have a wavelength suitable for detecting Mie scattering from particles for the determination of particle size. Examples of suitable excitation sources include UV light and visible light radiation sources, such as UV light and visible light lasers, LEDs and the like. By way of example, the excitation source 112 preferably operates in a wavelength of about 270 nm to about 410 nm, preferably about 350 nm to about 410 nm. A wavelength of about 270 nm to about 410 nm is chosen based on the premise that microbes and biological particles comprise three primary metabolites: tryptophan, which normally fluoresces at excitation wavelengths of about 270 nm with a range of about 220 nm - about 300 nm; nicotinamide adenine dinucleotide (NADH) which normally fluoresces at excitation wavelengths of about 340 nm (range about 320 nm - about 420 nm); and riboflavin which normally fluoresces at excitation wavelengths of about 400 nm (range about 320 nm - about 420 nm). In the case of bacterial endospores, dipicolinic acid (DPA) normally fluoresces at excitation wavelengths of about 400nm (range about 320nm-about 420nm). Preferably, however, the excitation source 112 has a wavelength of about 350 to about 410 nm. This wavelength ensures excitation of two of the three aforesaid primary metabolites, NADH, and riboflavin, and DPA" in bio-particles but excludes excitation of interferences such as from diesel engine exhaust and other inert particles such as dust or baby powder. Thus, in a preferred embodiment the present invention makes a judicial selection of the wavelength or wavelength range of the excitation source 112, which retains the ability of exciting fluorescence from NADH and riboflavin (foregoing the ability to excite tryptophan) while excluding the excitation of interferents such as diesel engine exhaust. This step is taken to reduce false alarms generated by diesel exhaust (which can be excited by short UV wavelengths such as 266 nm light.

In the system illustrated in Figures 2-6 a fluid sample, (e.g. environmental air or a liquid sample) is drawn into the system through an entrance nozzle 116 for particle sampling. Nozzle 116 is aligned with exit nozzle 117, allowing the particle stream to pass through the path of the electromagnetic radiation 114. Directly downstream from the laser beam is a Mie scattering particle-size detector. The Mie scattering particle-size detector includes a beam blocker 122 in front of a collimator lens 124 and a condenser lens 126 for focusing a portion of the light beam 114 scattered by particles in the sample stream onto a particle detector (not shown).

Off axis from, and preferably orthogonal to, the electromagnetic radiation 114, a pair of elliptical mirrors 130, 131 are placed around the particle-sampling region inverted and facing each other in such a way that the intersection of the incoming particle stream and the laser beam is at one foci of each mirror, while a fluorescence detector 132, 133 (for example, a photo-multiplier tube) occupies the other foci of each mirror. The elliptical mirrors are preferably placed out of plane of the Mie scattering optics, such that they are orthogonal to the Mie scattering optics. This design utilizes the fact that a point source of light emanating from or passing through one of the two foci of an ellipsoid will be focused onto the other. In this optical design, the elliptical mirrors 130, 131 concentrate the fluorescence signal from microbe and focus it onto the fluorescence detectors 133 and 132 respectively. Preferably, the fluorescence detectors are photo-multiplier tubes (PMTs). Optical filters 134, 135 may be placed in front of the fluorescence detectors to block the scattered light and pass the induced fluorescence.

The pair of elliptical mirrors form an enclosure around the particle detection area with openings for the nozzles 116, 117; the fluorescence detectors 132, 133; the electromagnetic radiation 114; and the Mie scatter cone (see Figure 5). As shown in the figures, the nearest focus of a given elliptical mirror will be at the intersection of the particle stream and the laser beam. The farthest focus of a given mirror will, as stated above, be at the fluorescence detector. Ideally, this opposite focal point will lie at the apex of the opposing elliptical mirror. This is the case if both ellipsoid are identical and if the distance between the foci is equal to one third of the length of the major axis of the ellipsoid.

The beam blocker 122 is designed to absorb, stop and/or contain non-scattered elements of the beam of electromagnetic radiation 114, , e.g. the laser beam, and may comprise light absorbent materials, such as vinyl, fluoroelastomers, metallic materials or the like, and/or geometries designed to collect and contain the radiation attached to a front surface of, for example, an optical element. Other features and considerations for the beam blocker 122 are disclosed in some of the earlier US Patents to Hamburger et al. listed above, and in PCT Application Serial No. PCT/US2006027638. Other features and considerations for the particle detector are disclosed in earlier commonly owned references, listed above, and the disclosures of which that are not inconsistent with the disclosure herein are incorporated herein by reference.

The present invention's use of Mie scattering also facilitates the placement of optical components for the detection of light induced fluorescence to concurrently examine individual particles for the presence of the metabolites NADH, riboflavin and other bio-molecules, which are necessary intermediates for metabolism of living organisms, and therefore exist in microbes and biological particles such as bacteria and fungi. If these chemical compounds exist in a bio-aerosol, they can be excited by photon energy and subsequently emit auto-fluorescence light which may be detected by an instrument based on the detection scheme outlined above. While this detection scheme is not capable of identifying the genus or species of microbes, and viruses may be too small and lack the metabolism for detection, this detection scheme's ability to simultaneously and for each particle determine the size of the particle and if it is biologic or inert indicates to the user the presence or absence of microbial contamination.

The double ellipsoidal mirror configuration has several advantages over the prior art. From Figure 1, it is apparent that much of the fluorescent signal is not captured by the ellipsoidal mirror of the prior art. The signal received by the detector is weak and difficult to amplify without also amplifying signal noise as well. With the additional ellipsoidal mirror and detector, however, the two signals received by the detectors can be compared so that a signal processor can distinguish the fluorescence signal from the noise.

In a comparative example, only one elliptical mirror is used. The elliptical mirror is rotated from its position according to the prior art (Figure 1) until it faces 90° from the light source and is orthogonal to the Mie scattering optics. This requires a repositioning of the fluorescence detector to align with the elliptical mirror. This orthogonal configuration allows the construction of a smaller light box. This design also allows optimization of the optics and signal collection by reducing optical path overlap. The elliptical mirror may be built with the light box as single unit. This embodiment improves upon the prior art by providing a robust and compact design.

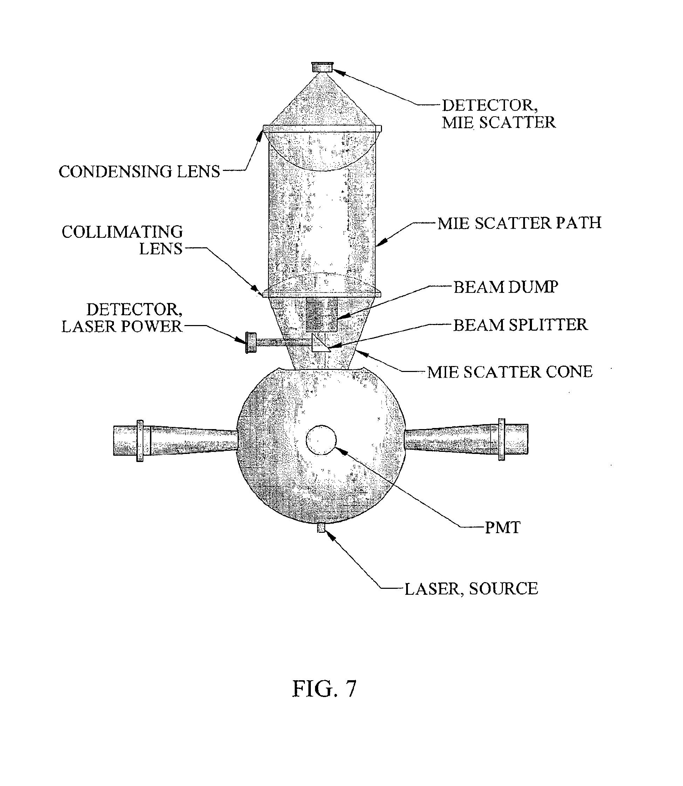

In other alternate embodiments, as shown in Figure 7, an optical element may be placed before the collimating lens to reflect a portion of the unscattered excitation beam to a detector, such as a photo detector, to measure excitation source power. In preferred embodiment the optical element reflects a portion of the unscattered bean 90 degrees, although other degrees of reflection may also be used. Examples of suitable optical elements include, but are not limited to, mirrors and the like.

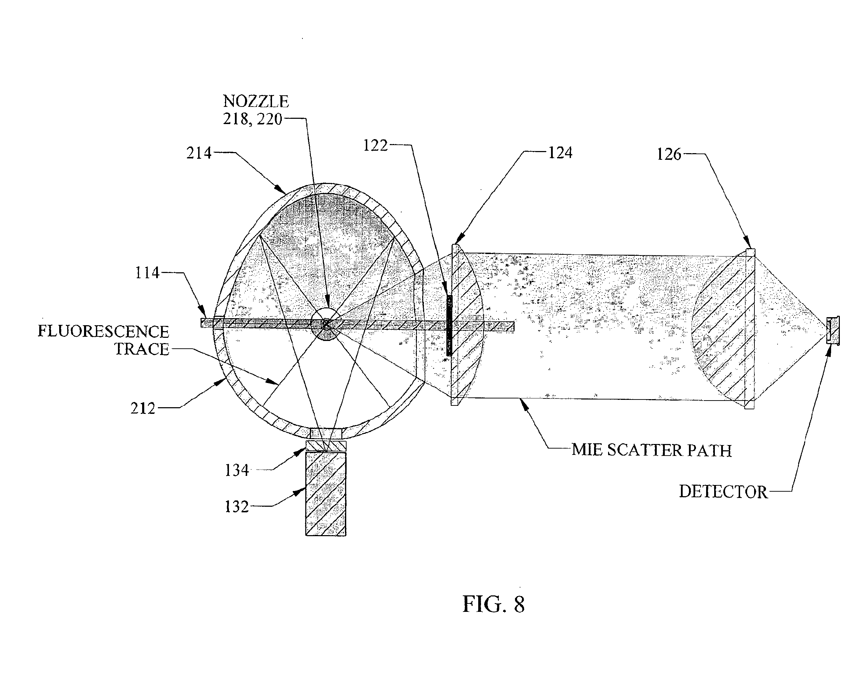

In a comparative example, a spherical mirror may be used with this single elliptical mirror embodiment to capture fluorescence signals that are escaping from the embodiments of the prior art. The spherical mirror 212 would be placed opposite the elliptical mirror 214 with the intersection of the particle stream and the electromagnetic radiation at its focal point. See Figure 8. The spherical mirror will reflect light back through the focal point and onto the elliptical mirror, which would then direct the light through an opening in the spherical mirror and into the fluorescence detector 216, which is shown in Figure 8 as a PMT. This embodiment captures a greater amount of the fluorescence signal without the cost of an additional fluorescence detector. Similar to the preferred embodiment, the spherical mirror 212 and the elliptical mirror 214 would form an enclosure 218, with openings only for the nozzles 220, the fluorescence detector 216, and the Mie scatter cone. The elliptical mirror and the spherical mirror could be extended to the point where the two surfaces would intersect to make this enclosure as complete as possible. In this alternative design the spherical mirror is positioned such that the intersection of the particle stream and the laser beam is at the center of curvature of the sphere.

In an alternative embodiment, a different configuration for the particle size detection system is contemplated. In this embodiment, the collimating and condensing lenses are oriented 90° from one another. A optical element, e.g. a mirror, is situated to direct the electromagnetic radiation from the collimating lens into the condensing lens. A beam blocker may be placed before the collimating lens as described above, or alternatively, the optical element (e.g. a mirror) allows direct radiation from the excitation source to pass through into a light dump positioned behind the optical element. This may be done by placing an appropriately sized opening in the optical element, for instance. Alternatively, the light dump may be replaced with another detector. This detector could measure the amount of light received to be compared with the output of the light source and the amount received by the other detectors. This configuration allows for the construction of a smaller light box and a more compact system design.

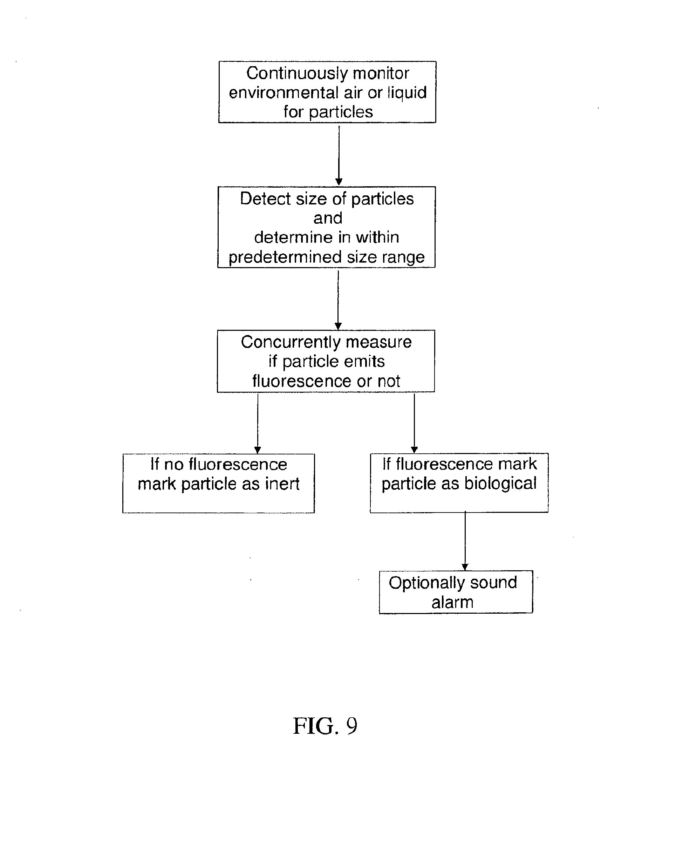

The functionality of the simultaneous particle sizing and fluorescence measurement scheme of the present invention is depicted in Figure 9. The principle of operation is as follows: an instrument continuously monitors the environmental air (or liquid) to measure the size of each individual airborne particle in real time and to concurrently determine whether that particle emits fluorescence or not. One or more thresholds are set for the fluorescence signal. If the fluorescence signal falls outside set parameters, the particle is marked inert. The fluorescence signal thresholds include one or more selected from fluorescence signal intensity, fluorescence intensity as a function of particle cross-sectional area or a function of particle volume. If the fluorescence signal threshold exceeds or falls within one or more set threshold levels, the particle is marked biological. The combined data of particle size and fluorescence signal strength will determine the presence or absence of microbes on a particle-by-particle basis. Other features and considerations for fluorescence signal thresholds are disclosed in commonly owned U.S. Patent Appl. No. 12/268,366 to Morrell et al.

It should be emphasized that the above-described embodiments of the present invention, particularly, any "preferred" embodiments, are merely possible examples of implementations, merely set forth for a clear understanding of the principles of the invention. Many variations and modifications may be made to the above-described embodiments of the invention without departing from the invention as defined in the appended claims. For example, the collimating and condensing lenses may be constructed as a single piece of the device. All such modifications and variations are intended to be included herein within the scope of this disclosure and the present invention and protected by the following claims.

## Claims

1. A method of differentiating biological particles from inert particles in a fluid sample which comprises drawing the fluid sample through an entrance nozzle (116), the entrance nozzle (116) being aligned with an exit nozzle (117) for allowing a particle stream to pass through a path of a focused beam of light (114) in a sample area; simultaneously measuring particle size and detecting intrinsic fluorescence from the particle stream wherein fluorescence intensity is measured and assigned a value, and including the step of classifying the particle as either inert or biological based on particle size and fluorescence intensity, and further including providing a pair of elliptical reflectors (130, 131) forming an enclosure around the sample area with openings for the entrance nozzle (116) and the exit nozzle (117), and wherein fluorescence is measured using two fluorescence detectors (132, 133), each of which receives fluorescence light captured by one of said two elliptical reflectors, wherein the pair of elliptical mirrors (130, 131) is located in a particle sampling region such that an intersection of the incoming particle stream and the beam of light (114) are at one foci of each ellipsoid, and one of said two fluorescence detectors (132, 133) lies at the other foci, wherein each of said fluorescence detectors (132, 133) produce a signal, said signals being compared, so that a fluorescence signal can be distinguished from noise, to determine relevant data, and wherein particle size is measured by the following steps:
sending the focused beam of light (114) through the sample, whereby portions of the beam of light are scattered at various angles by particles of various sizes present in the sample area, and unscattered portions of the beam of light remain unscattered;
blocking at least the unscattered portion of the beam of light (114) and limiting a range of particle sizes measured by arranging a beam blocking device (122) on an opposite side of the sample area; and
positioning a particle size detector in the light path after the beam blocking device (122) for detecting a portion of forward scattered light, and producing an output including information on the size of a single particle in the light path.

2. The method of claim 1, wherein size information of the particle is used to classify whether that particle is a microorganism.

3. The method of claim 1, wherein the two fluorescence detectors (132, 133) are positioned offaxis from the beam of light (114) for detecting intrinsic fluorescence from said same single particle, wherein said fluorescence detectors positioned orthogonal to said are beam of light (114).

4. The method of claim 1, wherein said fluorescence detectors (132, 133) are photo-multiplier tubes.

5. The method of claim 1, further comprising providing a collimating lens (124) on an opposite side of the sample from a source of said focused beam of light (114), providing an optical element for reflecting said portion of forward scattered light into a condensing lens (126) which focuses said portion of forward scattered light into said particle size detector, wherein said unscattered light is stopped by a beam blocker in front of the collimating lens (124) or passes through said optical element into a light dump.

6. The method of claim 1, further comprising providing an alarm unit for providing a warning signal when a particle within a predetermined size range is detected which also fluoresces.

7. The method of claim 1, wherein the focused beam of light has a wavelength in the range of 270 to 410 nm.

8. The method of claim 1, further comprising providing a processing unit for processing particle size distribution and particle fluorescence at a given time, and displaying a signal on an output device.

## Patentansprüche

1. Verfahren zum Unterscheiden von biologischen Partikeln von inerten Partikeln in einer Fluidprobe, umfassend das Ziehen der Fluidprobe durch eine Eintrittsdüse (116), wobei die Eintrittsdüse (116) mit einer Austrittsdüse (117) ausgerichtet ist, um zu ermöglichen, dass ein Partikelstrom durch einen Pfad eines fokussierten Lichtstrahls (114) in einem Probenbereich durchtritt; das gleichzeitige Messen einer Partikelgröße und Nachweisen einer intrinsischen Fluoreszenz aus dem Partikelstrom, wobei die Fluoreszenzintensität gemessen wird und dieser ein Wert zugeordnet wird, und enthaltend den Schritt des Klassifizierens des Partikels als inert oder biologisch auf Basis der Partikelgröße und Fluoreszenzintensität und darüber hinaus enthaltend das Bereitstellen eines Paares elliptischer Reflektoren (130, 131) zum Bilden einer Umgrenzung um den Probenbereich herum mit Öffnungen für die Eintrittsdüse (116) und die Austrittsdüse (117) und wobei die Fluoreszenz unter Verwendung von zwei Fluoreszenzdetektoren (132, 133) gemessen wird, wobei jeder davon Fluoreszenzlicht empfängt, das von einem der beiden elliptischen Reflektoren erfasst wird, wobei das Paar elliptischer Spiegel (130, 131) in einen Partikelprobennahmebereich angeordnet ist, so dass ein Schnittpunkt des eingehenden Partikelstroms und des Lichtstrahls (114) auf einem Brennpunkt jedes Ellipsoids liegt und einer der beiden Fluoreszenzdetektoren (132, 133) am anderen Brennpunkt liegt, wobei jeder der Fluoreszenzdetektoren (132, 133) ein Signal erzeugt, wobei die Signale verglichen werden, so dass ein Fluoreszenzsignal von Rauschen unterschieden werden kann, um relevante Daten zu bestimmen, und wobei die Partikelgröße mit den folgenden Schritten gemessen wird:
Senden des fokussierten Lichtstrahls (114) durch die Probe, wobei Abschnitte des Lichtstrahls über diverse Winkel von Partikeln diverser Größen gestreut werden, die im Probenbereich vorhanden sind, und ungestreute Abschnitte des Lichtstrahls ungestreut bleiben:
Blockieren zumindest des ungestreuten Abschnitts des Lichtstrahls (114) und Einschränken eines Bereichs von Partikelgrößen, die von einer Strahlblockierungseinrichtung (122) auf gegenüberliegenden Seiten des Probenbereichs gemessen werden; und
Positionieren eines Partikelgrößendetektors im Lichtpfad nach der Strahlblockiereinrichtung (122) zum Detektieren eines Abschnitts von vorwärtsgestreutem Licht und Erzeugen einer Ausgabe, die Informationen zur Größe eines einzelnen Partikels im Lichtpfad enthalten.

2. Verfahren nach Anspruch 1, wobei Größeninformationen des Partikels verwendet werden, um zu klassifizieren, ob dieser Partikel ein Mikroorganismus ist.

3. Verfahren nach Anspruch 1, wobei die zwei Fluoreszenzdetektoren (132, 133) außeraxial vom Lichtstrahl (114) angeordnet sind, um eine intrinsische Fluoreszenz aus dem gleichen einzelnen Partikel zu detektieren, wobei der Fluoreszenzdetektor orthogonal zum Lichtstrahl (114) angeordnet ist.

4. Verfahren nach Anspruch 1, wobei die Fluoreszenzdetektoren (132, 133) Photovervielfacherröhren sind.

5. Verfahren nach Anspruch 1, darüber hinaus umfassend das Bereitstellen einer Kollimatorlinse (124) auf einer gegenüberliegenden Seite der Probe von einer Quelle des fokussierten Lichtstrahls (114), Bereitstellen eines optischen Elements zum Reflektieren des Abschnitts von vorwärtsgestreutem Licht in einen Kondensor (126), die den Abschnitt von vorwärtsgestreutem Licht in den Partikelgrößendetektor fokussiert, wobei das ungestreute Licht von einem Strahlblocker vor der Kollimatorlinse (124) gestoppt wird oder das optische Element in eine Lichtfalle passiert.

6. Verfahren nach Anspruch 1, darüber hinaus umfassend das Bereitstellen einer Alarmeinheit zum Bereitstellen eines Warnsignals, wenn ein Partikel innerhalb eines vorab festgelegten Bereichs detektiert wird, der ebenfalls fluoresziert.

7. Verfahren nach Anspruch 1, wobei der fokussierte Lichtstrahl eine Wellenlänge im Bereich von 270 bis 410 nm hat.

8. Verfahren nach Anspruch 1, darüber hinaus umfassend das Bereitstellen einer Verarbeitungseinheit zum Verarbeiten einer Partikelgrößenverteilung und einer Partikelfluoreszenz zu einem bestimmten Zeitpunkt und Anzeigen eines Signals auf einer Ausgabeeinrichtung.

## Revendications

1. Procédé permettant de distinguer des particules biologiques de particules inertes dans un échantillon de fluide, qui comprend l'emportement de l'échantillon de fluide au travers d'une buse d'entrée (116), la buse d'entrée (116) étant alignée avec une buse de sortie (117) pour laisser un flux de particules traverser le trajet d'un faisceau de lumière concentré (114) dans une zone échantillon ; simultanément, la mesure de la granulométrie et la détection de la fluorescence intrinsèque émise par le flux de particules, dans lequel l'intensité de la fluorescence est mesurée et une valeur lui est attribuée, et comprenant l'étape de classement de la particule comme inerte ou comme biologique, selon sa granulométrie et l'intensité de sa fluorescence, et comprenant en outre la fourniture d'une paire de réflecteurs elliptiques (130, 131) formant une enceinte autour de la zone échantillon avec des ouvertures pour la buse d'entrée (116) et la buse de sortie (117), et dans lequel la fluorescence est mesurée au moyen de deux détecteurs de fluorescence (132, 133), chacun desquels reçoit une lumière de fluorescence capturée par l'un desdits deux réflecteurs elliptiques, dans lequel la paire de miroirs elliptiques (130, 131) est située dans une région d'échantillonnage de particules de sorte que l'intersection du flux de particules entrantes et du faisceau de lumière (114) se situe à un foyer de chaque ellipsoïde, et l'un desdits deux détecteurs de fluorescence (132, 133) se trouve à l'autre foyer, dans lequel chacun desdits détecteurs de fluorescence (132, 133) produit un signal, lesdits signaux étant comparés, de sorte qu'un signal de fluorescence puisse être distingué d'un bruit, pour déterminer des données pertinentes, et dans lequel la granulométrie est mesurée par les étapes suivantes :
l'envoi du faisceau de lumière concentré (114) à travers l'échantillon, moyennant quoi des parties du faisceau de lumière sont dispersées selon différents angles par des particules de granulométries diverses présentes dans la zone échantillon, et les parties non dispersées du faisceau de lumière restent non dispersées ;
le blocage au moins de la partie non dispersée du faisceau de lumière (114) et la limitation de la plage des granulométries mesurées par l'agencement d'un dispositif de blocage de faisceau (122) sur un côté opposé de la zone échantillon ; et
la mise en place d'un détecteur de granulométrie dans le trajet de lumière après le dispositif de blocage de faisceau (122) pour détecter une partie de la lumière dispersée vers l'avant, et produire une sortie comprenant des indications sur la granulométrie d'une seule particule dans le trajet de lumière.

2. Procédé selon la revendication 1, dans lequel les indications de granulométrie de la particule sont utilisées pour classer éventuellement la particule en question comme microorganisme.

3. Procédé selon la revendication 1, dans lequel les deux détecteurs de fluorescence (132, 133) sont placés de manière désaxée par rapport au faisceau de lumière (114) pour détecter la fluorescence intrinsèque émise par ladite seule et même particule, dans lequel lesdits détecteurs de fluorescence sont placés de manière perpendiculaire audit faisceau de lumière (114).

4. Procédé selon la revendication 1, dans lequel lesdits détecteurs de fluorescence (132, 133) sont des tubes photomultiplicateurs.

5. Procédé selon la revendication 1, comprenant en outre la fourniture d'une lentille de collimation (124) sur un côté opposé de l'échantillon par rapport à une source dudit faisceau de lumière concentré (114), la fourniture d'un élément optique pour réfléchir ladite partie de lumière dispersée vers l'avant dans une lentille de condensation (126) qui concentre ladite partie de lumière dispersée vers l'avant dans ledit détecteur de granulométrie, dans lequel ladite lumière non dispersée est arrêtée par un dispositif de blocage de faisceau devant la lentille de collimation (124) ou passe à travers ledit élément optique pour arriver dans un puits à lumière.

6. Procédé selon la revendication 1, comprenant en outre la fourniture d'une unité d'alarme destinée à fournir un signal d'avertissement en cas de détection d'une particule située dans une plage granulométrique prédéterminée et qui est également fluorescente.

7. Procédé selon la revendication 1, dans lequel le faisceau de lumière concentré a une longueur d'onde dans la plage de 270 à 410 nm.

8. Procédé selon la revendication 1, comprenant en outre la fourniture d'une unité de traitement destinée à traiter la distribution granulométrique et la fluorescence des particules à un instant donné, et à afficher un signal sur un dispositif de sortie.
